# EUROPEAN PATENT SPECIFICATION

(11) **EP 3 141 212 B1**
(45) Date of publication and mention of the grant of the patent: **06.11.2019**
(21) Application number: 15184635.9
(22) Date of filing: 10.09.2015
(51) Int. Cl.: A61F 2/01

(54) **AN EXTENDABLE, RETRIEVABLE ENDOVASCULAR ELEMENT**
ERWEITERBARES, WIEDERGEWINNBARES ENDOVASKULÄRES ELEMENT
ÉLÉMENT ENDOVASCULAIRE EXTENSIBLE RÉCUPÉRABLE

(43) Date of publication of application: 15.03.2017
(73) Proprietor: Endovascular Development AB, 111 56 Stockholm (SE)
(72) Inventor: Liungman, Krister, SE-753-13 Uppsala (SE); Bosaeus, Linus, SE-112 69 Stockholm (SE)
(74) Representative: Inspicos P/S

(56) References cited:
- WO-A1-01/21100
- WO-A2-02/43595
- US-B1- 6 939 361

## Description

The present invention relates to an assembly for introduction into a blood vessel and which has a maximum compression when attempted pulled out of the blood vessel. The present assembly may be used for fastening itself to a blood vessel while ensuring that the blood vessel is not exposed to excessive pulling force. It is an object to provide a guidewire fixator with increased safety preventing accidental repositioning of the guidewire while defining a maximum diameter of the fixator and thereby preventing exerting an excessive force to the blood vessel.

Endovascular elements or products of this type may be seen in e.g. US6991641, US6843798, US6695813, US7563272, US9039727, EP1676544, US8177807, US6599307, EP2745871, WO2012/065625, WO99/42059 and WO2005/037133.

WO0121100 discloses a medical filter that includes a radially expandable body having an opening in a proximal length thereof.

A first aspect of the invention relates to an endovascular assembly comprising:
- an elongate element having a proximal end and a distal end,
- a deformable element having a proximal slider and a distal slider configured to slide along the elongate element, the distal slider being positioned closer to the distal end than the proximal slider, the deformable element:
   - having a rest shape where a first distance exists between the proximal slider and the distal slider along a predetermined direction, the deformable element, in the rest shape, defining a first circumscribed circle with a first radius, in a plane perpendicular to the predetermined direction,
   - being extendable along the predetermined direction to a extended shape where a second distance exists between the proximal slider and the distal slider along the predetermined direction, the second distance exceeding the first distance, the deformable element, in the extended shape, defining a second circumscribed circle with a second radius, in the plane perpendicular to the predetermined direction, the first radius exceeding the second radius,
   - being compressible along the predetermined direction to a compressed shape where a third distance exists between the proximal slider and the distal slider along the predetermined direction, the first distance exceeding the third distance, the deformable element, in the compressed shape, defining a third circumscribed circle with a third radius, in the plane perpendicular to the predetermined direction, the third radius exceeding the first radius,
- a proximal stopper fixed or fixable in relation to the elongate element,
- a distal stopper fixed or fixable in relation to the elongate element,
   where:
   - the distal stopper is positioned closer to the distal end than the proximal stopper,
   - the proximal stopper is positioned closer to the distal end than the proximal slider, the proximal stopper being configured to prevent the proximal slider from moving closer to the distal end than the proximal stopper,
   - the distal stopper is positioned closer to the distal end than the distal slider, the distal stopper being configured to prevent the distal slider from moving closer to the distal end than the distal stopper, and
   - a distance, along the elongate element, between the proximal stopper and the distal stopper is no more than a predetermined distance being 0.95*the first distance.

In this context, an endovascular assembly is an assembly of which at least a portion, in this situation at least the deformable element, is configured to be provided inside a blood vessel of a person or animal.

An elongate element is an element having a longer dimension and at least one dimension perpendicular to this along which the element is shorter. In the present embodiment, the elongate element is at least 10 times, such as 100 times longer than its thickness (diameter if circular). Usually, the elongate element is flexible and possibly has a controllable portion the bending of which may be controlled in order to be useful for negotiating a path through the blood vessels of a person.

In many embodiments, the elongate element is a wire, such as a guidewire, which may be solid or have a hollow core, or a sheath, which may be an elongate, flexible, hollow tube through which gas, liquid or other sheaths or wires may be transported if desired. Often, the elongate element is sufficiently strong to withstand the pulling with at least 1N, such as 2, 3, 4, 5, 6, 7, 8, 9, 10 Newton or more. Also, the elongate element preferably is sufficiently stiff to transfer a pushing force applied at the proximal end to the distal end or the deformable element engaging the elongate element. In some embodiments, the elongate element is torsionally stable so that a rotation (around the longitudinal axis) of the proximal end will rotate also the distal end of the elongate element. In a preferred embodiment described below, the elongate element may be formed by multiple elongate elements.

In the present context, one end of the elongate element is a proximal end, which normally is the end operated by an operator when the other end, the distal end, is positioned within the blood vessel. Thus, the distal direction is a direction toward the distal end. Usually, the deformable element is at or close to the distal end. Mostly, the deformable element is closer to the distal end than the proximal end.

The deformable element may have any shape and may be provided or manufactured in any manner, as long as it can be extended along the predetermined direction and compressed along the predetermined direction.

In this context, the predetermined direction often is a direction of the blood vessel in which the deformable element is positioned during use. The predetermined direction may be a straight line, if the assembly is provided along a straight line e.g. on a table for inspection or test.

Often, the direction is an axis around which the deformable element is symmetric or at least substantially symmetric.

The deformation of the deformable element is the expansion or extension along the direction. In the extended state, the circumscribed circle is smaller than in the rest case and in the compressed state. Thus, by varying the longitudinal extent of the deformable element along the direction, the size thereof, in a plane perpendicular to the direction, may be varied.

This deformability may be obtained using oblong element(s), such as strains, wires, filaments or the like of metal, plastics, polymers, alloys or the like, fixed at one end to the proximal slider and at the other end to the distal slider. Oblong elements may be made by cutting elongate cuts in e.g. tubes. In some situations, multiple such tubes may be provided within each other. Oblong elements may also be used for forming an expandable/compressible braid or weave or a parachute/umbrella-shape which is usual in fixators, filters and the like.

When a distance between the proximal slider and the distal slider is reduced, the oblong elements will tend to bend more and thus extend further from an axis through the proximal slider and the distal slider. This is irrespective of whether the oblong element also extends around the axis or within a plane also comprising the axis.

Preferably, the deformable element has no oblong elements or the like extending solely in a plane perpendicular to the longitudinal axis, as such elements, unless extendable and contractible in their longitudinal direction, may prevent the variation desired in the circumscribed circle.

The oblong elements may be combined to form e.g. braids, which again may be basket-shaped braids having, in a cross section comprising the direction or axis, a central portion extending the farthest from the axis and two tapering portions at either end thereof which connect the central portion to the proximal slider and the distal slider, respectively.

Another type of deformable element may be a parachute-shaped element having a first set of struts extending from one of the proximal slider and the distal slider and to an outer portion configured to contact the blood vessel and a second set of struts extending from the other of the proximal slider and the distal slider to the outer portion. Often one set of struts support a filtering surface or the like, but this is not required according to the invention.

Usually, a circumscribed circle of the proximal slider and the distal slider, in the plane perpendicular to the direction, has a radius larger than or equal to the second radius, as it may be desired that the deformable element is capable of being collapsed to the diameter of the slider(s) even if carrying emboli or the like.

In this context, a slider is an element configured to slide along the elongate element. Sliding along may be sliding over the elongate element which then slides through a channel or hole in the slider. Alternatively, the slider may comprise an element through which the elongate element travels during the sliding. Alternatively, the elongate element could comprise a longitudinal slot wherein an element of the slider slides. The opposite set-up naturally is also possible.

The deformable element has a rest shape. In this context, the rest shape is a shape which the deformable element will maintain if not exposed to external forces, such as when lying on a plane, horizontal surface. The deformable element may have multiple rest shapes. In many situations, the rest state is obtained by deforming the deformable element into the desired rest shape and then treating the deformable element, such as using heat or chemicals, to retain this shape until forced or deformed into another shape.

In its rest shape, the deformable element has a first circumscribed circle. Preferably, this first circumscribed circle is defined by not the proximal slider nor the distal slider but a deformable portion, such as the above oblong elements (such as when forming a weave, braid, umbrella-shape or elongate, parallel struts), provided between the proximal slider and the distal slider, so that the widest portion of the deformable element is formed by the oblong elements. Preferably, the deformable portion also defines the third circumscribed circle when the deformable element is compressed to obtain the even larger circumscribed circle.

In the extended situation, where the deformable element defines the second circumscribed circle, this second circle may be defined by any of the deformable portion, the proximal slider and the distal slider. Thus, any of these parts may be the "widest" or "thickest" part of the assembly.

In this context, the predetermined direction is the direction along which the deformable element is extended/compressed. Usually, this direction is defined by the longitudinal element between the proximal slider and the distal slider, as the longitudinal element may provide the compressing/extending force or at least control the movement of the sliders during the compression/expansion.

A circumscribed circle, in this context, is the smallest possible circle within which all parts of the deformable element are provided, where the circle is defined in a plane perpendicular to the predetermined direction. Usually, the circumscribed circle will be the smallest circle over all of the longitudinal distance along the direction over which the deformable element is provided, so that the circumscribed circle describes the largest cross section or extent of the deformable element along its length.

Usually, deformable element is symmetric about the predetermined direction so that the circumscribed circles will have centres at positions in the plane corresponding to the elongate element, this direction or this axis, but this is not a requirement. The deformable element need not be rotationally symmetric.

It is seen that the difference between the rest position, the extended position and the compressed position may be seen in the distance between the proximal slider and the distal slider. The larger the distance, the lower may the circumscribed circle be. The rest position is seen between the compressed situation and the extended situation.

The proximal stopper and the distal stopper is/are fixed or fixable in relation to the elongate element. Thus, the stoppers are not slidable or translatable in relation to the elongate element but may be fastened or fixed in relation thereto via or through another element. Each stopper may be a separate part fixed to or in relation to the elongate element, or it may be an integral part of the elongate element, such as an expanded portion of the elongate element, a knot on the elongate element or the like.

When the proximal stopper is positioned closer to the distal end than the proximal slider, the proximal stopper is able to prevent the proximal slider from moving closer to the distal end than the proximal stopper. The same is the situation for the distal stopper and the distal slider. Thus, it is ensured that the elongate element is not accidentally pulled and released from the deformable element. In addition, the operation of the stoppers and sliders provides safety in that it allows an increase of diameter/circumscribed circle of the deformable element but only to a limit. Thereafter, the increase will stop to prevent traumatizing the blood vessel.

A stopper may prevent a slider from passing it by having a size or shape preventing the slider from sliding over it. In general, if the outer contour of the stopper does not fit within an inner contour of the slider, the slider may not be able to pass the stopper. In one example, however, the slider may have an inner thread fitting an outer thread of the stopper so that a simple translation will not be possible but a rotation of the stopper relative to the slider will allow the stopper to pass through the slider.

In the situation where the stoppers are circular in cross section and the slider has a circular opening therein, the diameter of the stopper should be larger than the inner diameter of the pertaining slider. The proximal stopper, however, may be desired smaller than the opening in the distal slider so that the proximal stopper may pass through the distal slider. Alternatively, the proximal stopper may also engage the distal slider. This is described further below. When the distance, along the elongate element, between the proximal stopper and the distal stopper is no more than a predetermined distance being 0.95*the first distance, it is ensured that there is a limit to how compressed the deformable element can be. Naturally, the predetermined distance may alternatively be selected as 0.9, 0.85, 0.8, 0.75 or even less, times the first distance.

When the deformable element is compressed, the distance between the distal slider and the proximal slider will decrease until this distance equals the predetermined distance between the two stoppers. A further pulling may result in a translation of the distal stopper and slider within the blood vessel, but due to the distance equalling the predetermined distance, the proximal stopper will engage the proximal slider so that this translation also results in the same translation of the proximal slider and thus of all of the deformable element. Thus, no increase in the circumscribed circle is seen. Instead, the deformable element may be displaced within the blood vessel.

In this context, the distance, naturally, is determined in the same manner during the rest shape, the compressed shape end the extended state. This distance may e.g. be between the portions or surfaces of the proximal and distal stoppers facing/engaging the proximal and distal sliders, respectively. Alternatively, other positions or parts of the stoppers, such as centres, may be used if desired. It is noted that elements may be provided between a slider and a stopper, which elements have the function of displacing/translating/replacing such engaging surfaces. In one example, the element may be a tube sliding along the guidewire and positioned between a slider and a stopper. Then, the real engaging, distance defining, surface is that of the tube and not the stopper/slider. A reason for providing such elements is that the same elongate elements (same stopper positions) and the same deformable element (with the same distance between the sliders in the rest position) may be used while different effective stopper (or slider) distances are obtained. It is noted that the addition of a small tube is much easier than manufacturing and storing different elongate elements with different stopper distances and/or different deformable elements with different slider distances. Thus, when the deformable element is provided in the blood vessel and has a shape equal to the rest shape or preferably slightly compressed in relation to the rest shape, a pulling of the elongate element will result in the distal stopper engaging the distal slider and thus a further compression of the deformable element, until the proximal stopper and the proximal slider engage, where after a further pulling will not further compress the deformable element. The predetermined distance may be selected to obtain a predetermined or desired maximum circumscribed circle and/or a maximum pulling force accepted until the deformable element is allowed or desired to move within the blood vessel to prevent that an excessive force is applied to the blood vessel during the pulling.

Thus, the deformable element may be slidable in relation to the elongate element and only engage the elongate element via the sliders. In that situation, the elongate element may be translated distally in relation to the deformable element when positioned in the blood vessel.

Then, the proximal stopper may in one embodiment be configured to pass the distal slider when moved distally. In that situation, the elongated element may be moved so far up into the blood vessel that both stoppers are positioned distally of the sliders, whereby the elongate element may potentially be placed as far up the blood vessel as desired. This passing may be effectuated by the proximal stopper having an outer shape (usually a diameter) fitting inside an opening in the distal slider, so that the proximal stopper may be translated through this opening.

In another embodiment, the proximal stopper is configured to engage the distal slider when moved distally. Thus, a distal movement of the elongate element will move the deformable element distally - now by the proximal stopper engaging the distal slider and thus transferring the movement to the deformable element. This may be used for moving the deformable element distally or for deployment from a delivery catheter, for example. Thus, the outer shape or contour of the proximal stopper does not fit within e.g. an inner shape or contour (such as a diameter) of an opening in the distal slider.

Usually, the elongate element will extend through the opening, if present, of the slider and thus be able to guide the stopper toward the opening/slider.

In this context, the inner/outer contours/surfaces/diameters may be defined by a number of shapes. Tubular (circular in cross section) are the preferred shapes, but triangular, squares, pentagons or the like, stars, pie-shapes or the like may also be used if desired. What is important is whether the proximal stopper engages the slider or not.

In one situation, the third distance is no more than 0.95*the first distance, such as no more than 0.9, 0.85, 0.8, 0.75, 0.7, 0.65, 0.6 or 0.5 times the first distance.

In an interesting embodiment, the elongate element is a single elongate element. In this context, a single elongate element is an element to which both stoppers are attached so that e.g. a distal pulling of the elongate element will pull both stoppers. The stoppers may be fastened or fixed to an outer surface of this elongate element and the same outer surface is available or exposed at the proximal end so that a pulling (accidental or on purpose) is obtained by engaging this outer surface.

As an alternative to the above single elongate element, another interesting embodiment is one wherein the elongate element comprises a first and a second elongate elements each having a proximal end and a distal end, wherein:
- the proximal slider is configured to slide along the second elongate element,
- the distal slider is configured to slide along the first elongate element,
- the proximal stopper is fixed or fixable in relation to the second elongate element, and
- the distal stopper is fixed or fixable in relation to the first elongate element, the assembly further comprising a locking element configured to lock the first elongate element to the second elongate element to maintain a predetermined relative longitudinal relation, along the predetermined direction, between the proximal and distal sliders.

The locking element may in principle be positioned anywhere in the assembly. Usually, it is desired that the locking element is operable from outside of the person, and therefore is preferred at the proximal ends of the elongate elements, or at least the proximal end of one of the elongate elements, but the actual locking may be performed at any position of the elongate elements.

In the locked situation, the predetermined relative longitudinal relation, along the predetermined direction, between the first and distal sliders is maintained. This longitudinal relation may define a distance between the stoppers along the direction, the advantages of uses of which are described further below. A longer distance may be used for retrieval of the system, as is described further below, and a shorter distance may be used for defining a maximum compression as described above.

Naturally, any or both of the proximal/distal slider may slide along both the first and second elongate elements if desired.

The locking may be a biasing of the elongate elements toward each other or the engaging of one elongate element with the other elongate element. Thus, the locking element may be an element, such as a clamp, configured to bias the elongate elements toward each other. Alternatively, the locking element may be a part of one of the elongate elements which enables that elongate element to fix itself in relation to the other elongate element. One elongate element may have a clamp, an expanded portion, a curved portion or the like configured to engage with the other elongate element. Other locking methods and elements may be external/internal threads on the elongated elements. A thread will prevent longitudinal movement unless it is rotated.

Alternatively, the locking element may be an element fastened to one elongate element, which element may engage or fix itself to the other elongate element to provide the locking. Naturally, the locking element may have multiple parts of which one is fixed to the first elongate element and another to the second elongate element.

Alternatively, a spacer element may be provided between e.g. projections of the first and second elongate elements, which spacer may prevent movement of one projection toward the other. The spacer may be a peel-away portion or layer, a coil, a clamp or the like. One projection may be the distal end of a longitudinal element and another projection may be a handle or outwardly extending portion of the other elongate element. The spacer may be a fixed spacer or a biasing spacer which may bias the two elongate elements toward a desired longitudinal relationship.

Preferably, the locking element is provided at the proximal end of at least one of the first and second elongate elements. In this manner, the locking element may be both positioned and operated outside of the patient. A locking element may be slightly bulky and thus not desired within the vasculature of the person. Also, if the locking element malfunctions, repair is much easier outside of the person. In one embodiment, the locking element has a circumscribed circle, in the plane perpendicular to the extent of one or both of the elongate elements, which is not larger than a circumscribed circle of one or both stoppers and/or that of the elongated element or deformable element. In this manner, other endovascular elements may be transferred from the proximal end of the elongate elements to the distal end thereof while the locking element is operating.

Thus, the locking may be an abutment, such as via a spacer element, an increased friction, a rotating/screwing action, a clicking action, a gluing, a welding/soldering, a bayonet locking,
In one situation, the first and second elongate elements are co-extending at at least a portion of the lengths thereof, the locking element being positioned so as to lock the first elongated element to the second elongate element at a position within the portion of the lengths.

When the elongate elements co-extend, locking of one in relation to the other is rather simple, and the controlling of a relative extent of one in relation to the other is simpler, as this may be determined at the portion where they co-extend.

Thus, there may be portions of the length of one of both of the elongated elements where they are not co-extending and where they may take different routes within the vasculature of the person or may exit the person at different portions.

The first and second elongated elements may co-extend, preferably where one extends within the other or both extend inside a third element, where the adaptation of the relative longitudinal relation between the first and second elongated elements may be the translation of one in relation to the other in the portion where they co-extend.

That the two elongate elements co-extend means that at least along this portion of the length of the two elongate elements, they are in the vicinity of each other, where "vicinity" may be within 5% of the length of the longest of the two elongate elements.

As mentioned, one manner of providing co-extending, elongate elements is to have both elements extend within a common, elongate element such as a tube or sheath having one or more channels wherein the co-extending elongate elements extend. In one situation, the two elongate elements may be wires extending within a common sheath. In another situation, one elongate element extends within the other elongate element which again extends within a third elongate element.

However, it is presently preferred that, at the portion of the lengths, the first elongate element extends within the second elongate element. The first elongate element may then exit the second elongate element at the proximal end thereof in order for the two elongate elements to be operable or handled at their respective proximal ends. Also, the first elongate element may exit the second elongate element at the distal end thereof and the distal stopper be positioned at the exposed, distal end of the first elongate element. Then, the first elongate element may be inside the second elongate element for the full length of the second elongate element.

In one situation, as mentioned above, the first elongate element extends within the second elongate element at the portion of the lengths.

In one situation, the locking element is provided at the proximal end of at least one of the first and second elongate elements.

Naturally, the assembly may further comprise additional elements or features, such as an engagement element at or in the proximal slider, which engagement element may be engaged by a retrieval element configured to engage the proximal slider and pull the proximal slider and the remainder of the deformable element into a catheter. Additionally or alternatively, the deformable element may comprise an element or a surface configured to filter emboli from a blood stream in the blood vessel or to occlude the blood vessel to prevent any blood flow therein. The present assembly may additionally be used for defining a position for drug delivery where the drug may be transported to the position through one or both elongate elements. Also, the assembly may be used for indicating a position in a blood vessel. The assembly, such as the deformable element, may have a recognizable element, such as a radiopaque marker, which may be seen in fluoroscopy or in an x-ray device.

In one situation, the assembly further has an element configured to define a minimum distance, along the elongate element, between the proximal stopper and the distal stopper. Thus, the minimum distance may be set according to e.g. the size or type of blood vessel or which other tasks are performed in the blood vessels of the person, such as which other tasks are performed using (such as over) the elongate element.

As mentioned above, the minimum distance defines the largest compression possible and thus aids in defining a maximum force transferred from a pull in the elongate element to the blood vessel.

This element may be an element preventing the proximal and distal stoppers from moving closer to each other than the minimum distance during translation of one elongate element in relation to the other. This element may simply be an oblong slider having a desired length and sliding on or along one or more of the elongate elements between the stoppers. The element may be fastened or form a part of a slider if desired. When the locking element is used, the locking element may act to prevent a longitudinal movement of the elongate elements increasing the distance between the stoppers where the preventing element may prevent longitudinal movement decreasing the distance between the stoppers.

An example to understand the invention relates to a method of operating the assembly of the first aspect, the method comprising:
1. providing the deformable element in a blood vessel of a person, so that it engages the blood vessel,
2. after step 1, pulling the elongate element with increasing pulling force in a direction out of the blood vessel to reduce a distance between the proximal and distal sliders and thus increasingly compress the deformable element to increase a radius of a circle circumscribing the deformable element, until a distance, along the elongate element, between the proximal and distal sliders reaches the predetermined distance,
3. after step 2, the proximal stopper preventing the proximal slider from moving closer to the distal stopper so that increasing pulling force results in no further increase in the radius of the circumscribing circle.

In this context, the operation of the assembly may be the use thereof in the process of an endovascular procedure. The present assembly may be used for securing the elongate element (i.e. a part thereof) in a particular blood vessel in order to use the elongate element for guiding other endovascular elements (such as balloons, stents, grafts or the like) to the blood vessel or along a route in the blood vessels defined by the elongate element. Alternatively, the deformable element may be used for filtering or occluding as described above.

The providing of the deformable element in the blood vessel usually comprises providing also a part of the elongate element and the deformable element in the blood vessel. This introduction usually is via an arterial puncture as is usual in the art.

Deployment of an endovascular element into a blood vessel usually is the providing of the element inside a deployment or delivery catheter which is guided to the blood vessel, where after the element is deployed therefrom to perform the desired function.

Usually, the deformable element is provided in a blood vessel so that it is at least slightly compressed from its rest shape to be at least slightly biased against the vessel wall to maintain its position. The amount of compression may be very small. Preferably, on the other hand, the circumscribed circle of the deployed deformable element does not exceed that obtained when the distance between the proximal and distal stoppers is the predetermined distance.

In one example in step 3, a distance between the proximal slider and the first portion reaches a third distance being no more than 0.95*the predetermined distance, such as no more than 0.9, 0.85, 0.8, 0.75, 0.7, 0.65, 0.6, 0.55, or 0.5 times the predetermined distance.

Another example to understand the invention relates to a method of operating the assembly of claim 7, the method comprising:
1. providing the deformable element in a blood vessel of a person, so that it engages the blood vessel,
2. activating the locking element to lock the first elongate element to the second elongate element so that a predetermined distance exists between the first and second stoppers,
3. after step 1, pulling the elongate element with increasing pulling force in a direction out of the blood vessel to reduce a distance between the proximal and distal sliders and thus increasingly compress the deformable element to increase a radius of a circle circumscribing the deformable element, until a distance, along the elongate element, between the proximal and distal sliders reaches the predetermined distance,
4. after step 2, the proximal stopper preventing the proximal slider from moving closer to the distal stopper so that increasing pulling force results in no further increase in the radius of the circumscribing circle.

In one example in step 4, a distance between the proximal slider and the first portion reaches a third distance being no more than 0.95*the predetermined distance, such as no more than 0.9, 0.85, 0.8, 0.75, 0.7, 0.65, 0.6, 0.55, or 0.5 times the predetermined distance.

In general, pulling the elongate element of the deployed assembly with increasing pulling force in a direction out of the blood vessel will force the distal stopper proximally, which will provide a proximally directed force to the distal slider and thus a part of the deformable element engaging the vessel wall. This will reduce a distance between the proximal slider and the distal slider and thus increasingly compress the deformable element to increase a radius of a circle circumscribing the deformable element. This will continue with increasing force, until a distance, along the elongate element, between the proximal and distal stoppers reaches the predetermined distance, whereby the proximal stopper will engage the proximal slider. After that, increasing the pulling force will not result in a further increase in the radius of the circumscribing circle. Instead, the deformable element is translated proximally within the blood vessel.

In one situation, the method may also comprise a step of pushing the elongate element further into the blood vessel, whereby the distal stopper may be transferred further into the blood vessel.

In that situation, the method may comprise a step of pushing the elongate element in a direction into the blood vessel (i.e. opposite to the pulling direction) where the proximal stopper passes the distal slider and is subsequently moved further into the blood vessel. In that situation, the elongate element may be transferred as far up the blood vessel as desired. In another situation, the method comprises a step of pushing the elongate element in a direction into the blood vessel (i.e. opposite to the pulling direction) comprises the proximal stopper engaging the distal slider, where after the distal slider, upon further pushing, is moved further into the blood vessel. As described above, this may be used for repositioning the deformable element or for deployment thereof.

In one situation, the assembly further comprises the above described element configured to define a minimum distance, along the elongate element, between the proximal stopper and the distal stopper. Thus, further compression/expansion may be prevented. As described above, this element may be an element provided in or at the deformable element, sliding on or at the elongate element(s) or may be an element operated or operable from outside of the person.

In one example in step 3, a distance between the proximal slider and the first portion reaches a third distance being no more than 0.95*the predetermined distance, such as no more than 0.9, 0.85, 0.8, 0.75, 0.7, 0.65, 0.6, 0.55, or 0.5 times the predetermined distance.

In one aspect the method further comprises the step of, subsequent to step 4, retrieving the deformable element by:
5. deactivating the locking element,
6. increasing the distance between the first and second stoppers,
7. translating a retrieval catheter relative to the deformable element to transfer the deformable element into the retrieval catheter, and
8. retrieving the retrieval catheter from the blood vessel.

Thus, initially, the locking element is de-activated to allow relative movement of the first and second elongate elements so that the distance between the stoppers may be altered.

This distance then is increased to allow the deformable element to extend and thus reduce in diameter of the circumscribed circle. The deformable element will seek toward its rest shape even though it may not be able to reach the rest shape due to the interaction with the blood vessel.

The retrieval catheter may be any type of hollow element which may be moved to a position in the vicinity of the deformable element. Usually, the hollow element is translated along the elongate elements to use the elongate elements to guide the retrieval catheter to the right position and so as to be able to use the elongate elements (see below) to force the deformable element into the retrieval catheter.

The translating of the retrieval catheter in relation to the deformable element usually is a pushing of the retrieval catheter and the pulling of the second elongate element. This pushing/pulling will pull the proximal slider into the retrieval catheter and will then act to gently deform and extend the deformable element in order for it to fit into the retrieval catheter. Finally, the distal slider will follow the deformable portion and the proximal slider, where after the deformable element is fully provided inside the retrieval catheter which may then be removed from the blood vessel.

The method may also comprise the deformable element, when deployed in the blood vessel, filtering emboli from a blood stream in the blood vessel or the deployed deformable element occluding a blood flow in the blood vessel.

In the following, preferred embodiments of the invention will be described with reference to the drawing, wherein:
- figure 1 illustrates a first embodiment of an assembly according to the invention where the deformable element is in its rest shape,
- figure 2 illustrates the first embodiment of figure 1, where the deformable element is compressed,
- figure 3 illustrates a second embodiment of an assembly according to the invention where the deformable element is in its rest shape,
- figure 4 illustrates the second embodiment of figure 2 where the deformable element is compressed,
- figure 5 illustrates the second embodiment of figure 2, where the distal stopper is pushed distally,
- figures 6-8 illustrate one manner of deploying an assembly according to the invention within a blood vessel,
- figures 9-12 illustrate a manner of retrieving an assembly according to the second embodiment of the invention,
- figures 13 and 14 illustrate different manners of providing an assembly suitable as an endovascular filter,
- figure 15 illustrates an embodiment where the elongate elements co-extend within a third elongate element, and
- figures 16 and 17 illustrate an embodiment where a stopper has an outer thread and a slider an internal thread.

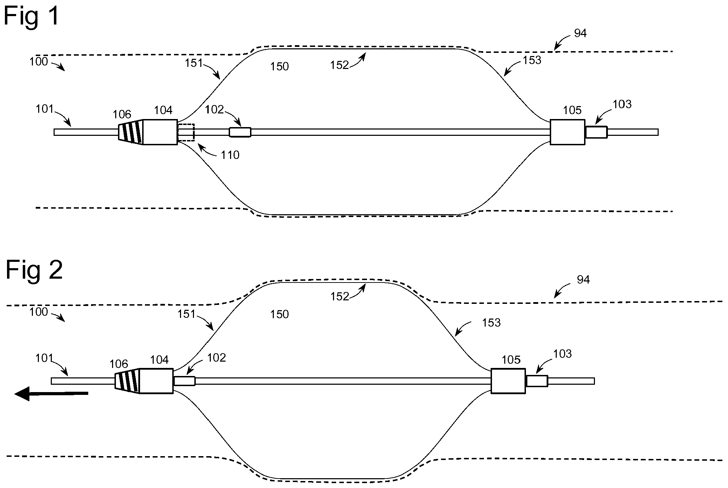

In figure 1, an assembly 100 is illustrated having an elongate guidewire 101 having a proximal end 90 and a distal end 92. Stoppers 102 and 103 are attached to the guidewire 101 at positions to be described further below.

In the present context, a "guidewire" may be any type of elongate element. The "guidewire" may be solid or hollow, be defined by one or more strands/wires, wound/braided/coiled or not, and/or as a tube, such as an extruded tube.

A sheath, in this context, is an elongate, hollow element through which a guidewire may be guided if desired. Also or alternatively, the sheath may be used for transporting liquid/gas.

The sheath may be formed as a tube, a hypotube, a coil or the like, and the sheath may comprise one or more channels therein for guiding a guidewire/liquid or the like.

The guidewire and sheath may or may not have a desired shape, such as by use of a memory shape material, such as an alterable shape as known for negotiating the way through blood vessels.

Any one of the guidewire and sheath may have a hydrophilic surface. Also, a soft, atraumatic tip may be provided to one or both of the guidewire and sheath to aid in guiding of the assembly within the blood vessel(s).The guidewire 101 travels through a deformable element 150 having sliders 104 and 105 at either end, an engaging surface 152 configured to engage a blood vessel 94 wherein the deformable element 150 is deployed. Between the engaging surface 152 and the sliders 104/105, tapering elements 151/153 are provided.

The deformable element 150 may have multiple functions in the blood vessel 94. One function is that of a filter configured to allow blood flow there through but to retain embolic material from e.g. surgery or angioplasty performed upstream of the position of the filter. Another function is that of occluding the blood vessel, where the deformable element may have a surface or an element configured to prevent blood through the deformable element. A further or additional function may be that of fixing itself to the blood vessel to fix the position or the presence of the guidewire in the blood vessel. Literature describing these different types of uses or functions are described further above.

The deformable element may be provided or generated in a number of manners. In one situation, the deformable element is formed as a braid of a number of braided, elongate elements, such as wires (e.g. NiTiNol or other biocompatible materials). Alternatively, the deformable element may be formed by cutting a tube, such as a hypotube, along elongated lines whereby, upon compressing the hypotube along the longitudinal direction, the resulting, elongate elements will "bulge" outwardly and thus form a deformable element.

The deformable element may be basket-shaped as illustrated or rather shaped as a parachute with support wires (from which the person hanging in the parachute hangs) and having at one end the "top" of the parachute, and at another end, an element where the support wires are attached. The parachute will typically engage the blood vessel at its outer parts.

Different portions of the deformable element may have different properties. Thus, if the deformable element is for use as e.g. a filter, one or both of the tapering portions 151/153 may be provided with the filtering properties (number or size of openings required for filtering) where the engaging surface may have other properties, such as for optimal engagement with the blood vessel. In the parachute example, the parachute-portion may have the filtering properties and the "wires" other properties.

Preferably, the deformable element is self expanding so that it, when unstressed, reaches a rest shape having a first distance between the sliders 104 and 105 and a first circumscribed circle in a plane perpendicular to the straight line through the sliders 104/105 - along which the guidewire 101 extends.

Usually, the deformable element of the assembly is selected to have a rest shape equal to or slightly larger than the diameter/dimensions of the blood vessel so that the deformable element in the rest shape will engage the inner side of the blood vessel. Self expansion may be obtained by providing the deformable element with the desired shape (such as on a mandrel) and subsequently treating the deformable element, such as by a heat treatment, to permanently shape the deformable element to this desired shape.

It is seen that the distance between the stoppers 102 and 103 in figure 1 is smaller than the distance between the sliders 104/105. Thus, when the guidewire 101 is pulled (to the left) outwardly of the blood vessel, the stopper 103 will initially contact the slider 105 and act to force the slider 105 in the same direction. This will act to compress the deformable element 150 longitudinally and thus, if the deformable element is adapted to do so, increase the diameter of the deformable element. This increases the engagement between the deformable element and the blood vessel, and this is the situation seen in figure 2.

In figure 2, the guidewire 101 has been pulled to the degree that the proximal stopper 102 engages the proximal slider 104. Clearly, pulling the guidewire 101 harder will now act to attempt to translate the assembly inside the blood vessel. Pulling the guidewire 101 harder will not increase the diameter of the circumscribed circle. Thus, pulling the guidewire 101 harder will not increase the circumscribed circle, and thus the blood vessel, further. The distance between the stoppers 102/103 thus aid in limiting a maximum force exertion of the blood vessel.

The expansion of the deformable element, thus, is desired in this embodiment. Extension may be allowed when the deformable element does not have an element limiting the diameter (such as a diameter of the circumscribed element) or circumference of the deformable element. A limiter of this type may be a non-stretchable band extending along the circumference of the deformable element in a plane perpendicular to the "longitudinal" direction also followed by the guidewire 101.

For example, when the deformable element comprises a braid of wires or other elongate elements extending at an angle different from perpendicular to the longitudinal direction, compression of the deformable element is possible. These wires or other elements may extend within a plane also comprising the longitudinal direction or around the longitudinal direction while having a projection along the longitudinal direction.

The deformable element may, in addition to, or instead of, such elongate elements, have additional components for providing particular properties to the deformable element. In one example (see also further below), the deformable element may comprise a sheet-like material which has openings and which forms a filtering or occluding surface. In that situation, the sheet-like material may be sufficiently pleated or sufficiently expandable/extendable to allow the compression of the deformable element.

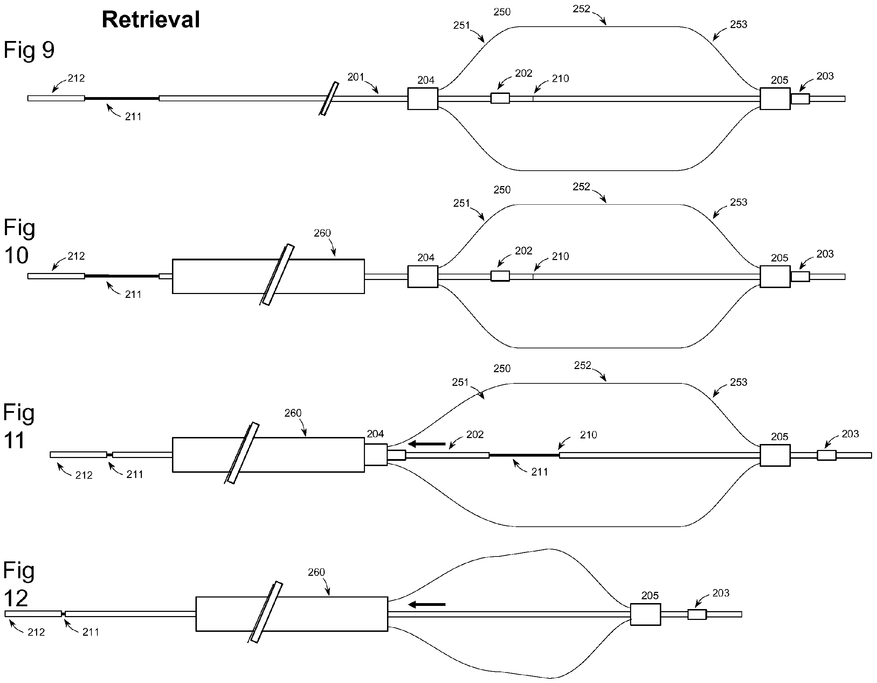

In figures 1 and 2, a thread 106 is illustrated. This thread may be used for deployment and especially retrieval of the deformable element 150 such as by using a retrieval catheter engaging the thread 106 to pull the deformable element into a retrieval catheter via which the deformable element may be removed from the blood vessel. In figures 9-12, another retrieval method is described.

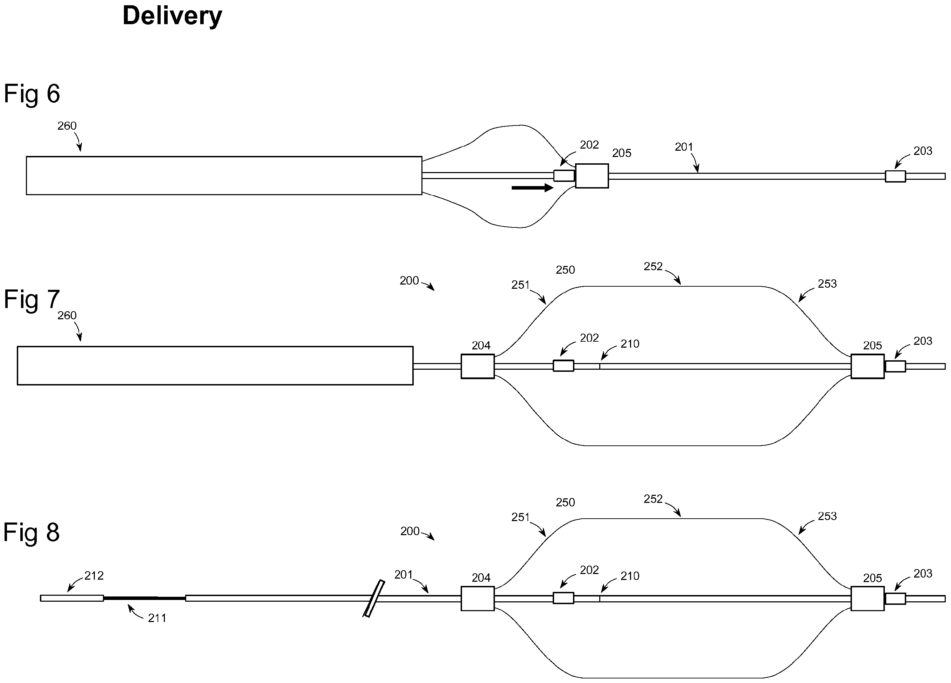

It is seen that when providing two sliders, the guidewire 101 may be translated or pushed further into the blood vessel. The stopper 102 may, upon pushing, engage the slider 105 (see the equivalent operation in fig. 6 and as described below) whereby the deformable element may be pushed further into the blood vessel or push the element 150 out of a delivery sheath. Alternatively, the stopper 102 may be adapted to be translated through the slider 105 (either by e.g. having an outer diameter smaller than an inner diameter of the slider 105 or by having an outer thread adapted to an inner thread of the slider 105 so that rotation may bring the stopper 102 distally of slider 105), so that the guidewire 101 may be translated as far into the blood vessel as desired without affecting the position of the deformable element.

In figure 1, a tube or slider 110 is illustrated which may be used to alter the interaction or method of the assembly. The tube/slider 110 alters the effective distance between the stoppers 102/103 in that it effectively alters the position of the surface engaging the slider 104 from the proximal surface of the stopper 102 to that of the tube 110. Thus, it is seen that the distance between the sliders 105/104, when both being engaged by the stoppers 102/103 - now via the tube 110 - is higher. Thus, a lower compression is allowed by the element 150 before the compression is stopped.

The use of a tube/slider 110 may thus be used for changing the characteristics of an assembly. An alternative would be to change the positions of the stoppers and/or change the shape of the element 150 which is a more expensive and labour consuming step.

Naturally, the element 110 may optionally or additionally be positioned between the distal slider 105 and the distal stopper 103.

Providing a set of assemblies as seen in figure 1 and one or more tubes, of different lengths, would equate providing different assemblies with different distances between the stoppers and/or different shapes of elements 150- such as different distances between the sliders in the rest position.

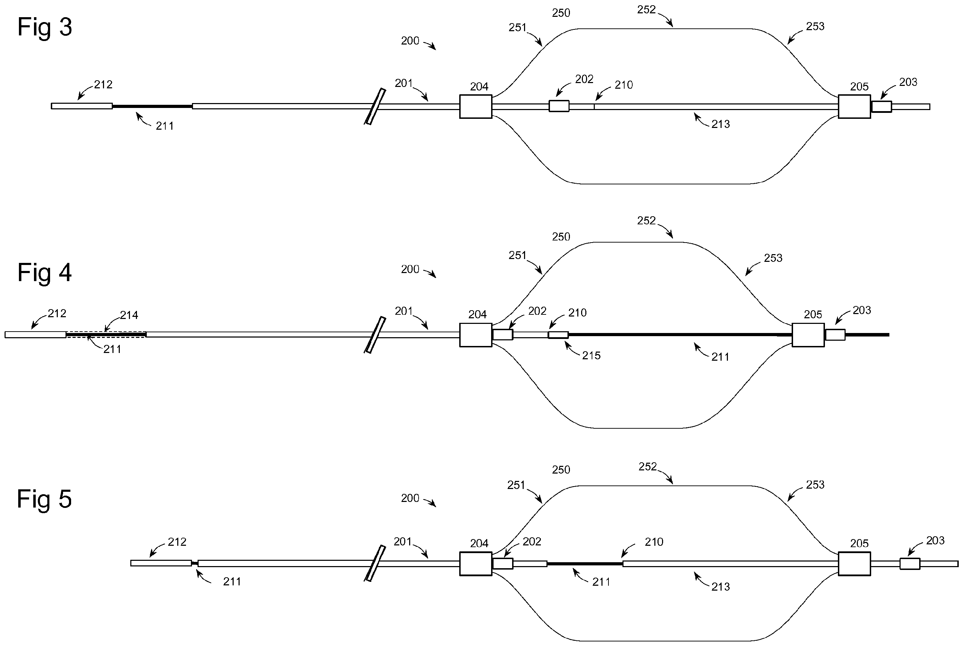

In figures 3-5, a second embodiment of an assembly 200 according to the invention is illustrated. This embodiment has two stoppers 202/203 and the deformable element 250 with sliders 204/205, an engagement portion 252 and tapering portions 251/253.

In the assembly 200, an inner guidewire 211 is provided co-extending within an outer sheath 201, where the stopper 203 is fixed to or in relation to the inner guidewire 211 and the stopper 202 is fixed to or in relation to the sheath 201. It is noted that this co-extending is not absolutely required and that the guidewire and sheath may be provided not inside each other and be guided to the deformable element 250 along two different routes, but this is usually very inconvenient.

In one embodiment, the set of guidewire and sheath may be a movable core guidewire as is known in the art.

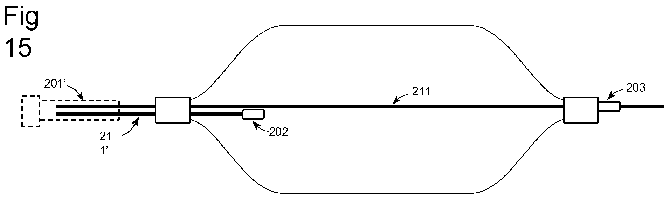

In figure 15, as an alternative, two elongate elements 211 and 211' extend within a common sheath 201'.

Thus, when the guidewire 211 is pushed distally (to the right) in relation to the sheath 201, the stopper 203 is moved distally in relation to the stopper 202, thus increasing the distance between the stoppers 202/203.

Also, when the guidewire 211 is pulled proximally (to the left) in relation to the sheath 201, the stopper 203 is moved proximally in relation to the stopper 202, thus decreasing the distance between the stoppers 202/203.

Thus, by operating the guidewire 211 in relation to the sheath 201, the distance between the stoppers 202/203 may be defined, whereby the limits of the expansion of the deformable element 250 may be defined. Thus, the above distance being smaller than the distance corresponding to the rest position of the deformable element 250 may be selected. In fact, an indication may be provided on the guidewire or sheath illustrating this particular distance or a desired distance smaller than this particular distance.

Pulling the guidewire 211 and sheath 201 (not altering the distance between the stoppers) will, as is described above, firstly increase the size perpendicular to the longitudinal axis of the deformable element 250 but will, when the stopper 202 engages the slider 204, stop increasing the size of the deformable element.

The distance between the stoppers 202 and 203 thus defines a maximum engagement force exerted to the blood vessel when pulling the guidewire/sheath.

In figures 3 and 5, an optional element 213 is illustrated which is connected to the stopper 203 and which engages the sheath 201 (but it could alternatively engage the stopper 202, slider 205 or slider 204). This element 213 defines a minimum distance between the stopper 203 and the stopper 202, when it engages, at point 210, the sheath 201. The element 213 could also engage the slider 204 if desired.

Clearly, the element 213 need not be attached to any of the sliders or stoppers and may simply freely translate between such elements. The element 213 may engage or define a minimum distance between any of, on the one side, the stopper 202, the slider 204 and the sheath 201 and, on the other side, the slider 205 and the stopper 203 or an element fixed in relation to any of the two.

An advantage of the element 213 is that the minimum distance is easily obtained, as the handle 212 may be pulled until the element 213 engages and thus provides a resistance to further pulling.

In figure 4, an alternative is illustrated where the element 213 has been replaced by a stopper 215 fixed to the guidewire 211.

The sheath 201 and guidewire 211 may be locked to each other at different positions. This lock may be a biasing/pressing, a snap fitting or any other type of lock. Also or alternatively, the guidewire 211 may have an outer thread engaging an inner thread in the sheath 201, so that a relative rotation may transport the guidewire 211 distally or proximally in relation to the sheath 201.

Another type of lock is indicated in figure 4, where an element 214 abuts the handle 212 and the proximal end of the sheath 201 so as to prevent the guidewire 211 from being translated distally in relation to the sheath 201. This lock 214 may be a clamp, a coil or other element, such as a peel-away portion of the sheath 201 or a separate peel-away portion. This lock thus may bias the handle 212 and thereby the guidewire 211 proximally in relation to the sheath 201. The lock 214 may cooperate with the above element 213 or stopper 215 to block translation of the guidewire 211, relative to the sheath 201, in both directions.

Different indications on the guidewire may be used for indicating the distance between the stoppers, the maximum outer diameter of the deformable element or the maximum force exerted on the blood vessel when the guidewire/sheath is pulled. Additionally or alternatively, the above element 213 or stopper 215 may be used for providing a tactile feedback of when the minimum distance is reached.

In figures 6-8, delivery of an assembly is described. The figures are illustrated with the assembly of figures 3-5, but the assembly of figures 1 and 2 may equally well be used for at least some of the operations.

In figures 6 and 7, a delivery catheter 260 is illustrated inside which the deformable element 250 is provided during insertion into the blood vessel. The insertion may be guided by the guidewire 211 and/or sheath 201 if desired.

When positioned correctly in the blood vessel, the guidewire/sheath is pushed distally, whereby the stopper 202 engages the slider 205 and thus forces the deformable element 250 out of the delivery catheter 260 (figure 6).

When fully delivered (figure 7), the deformable element 250 may function as described above, and the delivery catheter 260 may be removed by translation along the guidewire/sheath out of the person, where after (figure 8), the assembly is as described above.

In figures 9-12, retrieval of an assembly as described in relation to figures 3-5 is illustrated. The retrieval is performed in a retrieval catheter 260 which may be the same catheter as the above delivery catheter. The deformable element 250 may be collapsed and provided inside the retrieval catheter.

Initially (figure 9), the assembly is in the usual state in the blood vessel. Then, the retrieval catheter 260 is inserted (figure 10) and the guidewire 211 pushed distally to increase the distance between the stoppers 202/203 to allow the deformable element 250 to extend and thus be compressed (figure 11).

Then, the sheath/guidewire are pulled (together as they are locked to each other; the default situation) to force the stopper 202 in engagement/abutment with the slider 204 and pull the slider 204 and thus the element 251, then the engagement part 252 (figure 12) and subsequently the remainder of the deformable element 250 and the stopper 203 in to the catheter 260 which may subsequently be removed from the blood vessel.

Clearly, the radial compression of the deformable element 250 requires the deformable element to be elongated or extended, which is made possible by the increased distance between the stoppers 202/203.

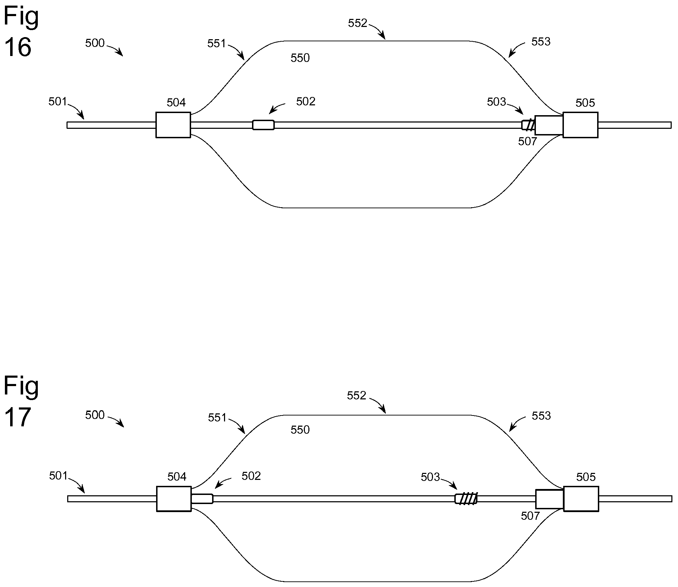

In figures 16 and 17 an embodiment is illustrated where the distal stopper 503 has an outer thread and the distal slider 505 of the deformable element 550 has a matching internal thread 507. Again, the deformable element has tapering portions 551 and 553 and an engaging portion 552 as well as a proximal slider engaging a proximal stopper 502 of the guidewire 501.

In figure 16, the threads of the stopper 503 and the slider 505 engage and rotation of the guidewire 501 in one direction may bring the stopper 503 distally of the slider 505. Rotation in the opposite direction will bring the stopper 503 proximally of the slider 505 as is seen in figure 17.

Thus, the distal stopper 503 may be brought into the position distally of the distal slider 505 so as to perform the above two-stopper function of limiting the extension of the deformable element 550 during pulling of the guidewire 501. Also, the stopper may be brought into the position proximally of the slider 505 to allow the deformable element to extend during pulling of the guidewire. In that manner, the deformable element is allowed to be pulled (see figures 9-12) into a catheter for removal of the deformable element from the blood vessel.

In general, the deformable element 150, 250, 350, 450, 550 may be used for a plurality of purposes. In one situation, the main purpose of the deformable element is merely to fasten itself to the blood vessel to prevent accidental removal of the guidewire from the blood vessel, such as in the situation where the guidewire is to be used for guiding other endovascular elements to the blood vessel.

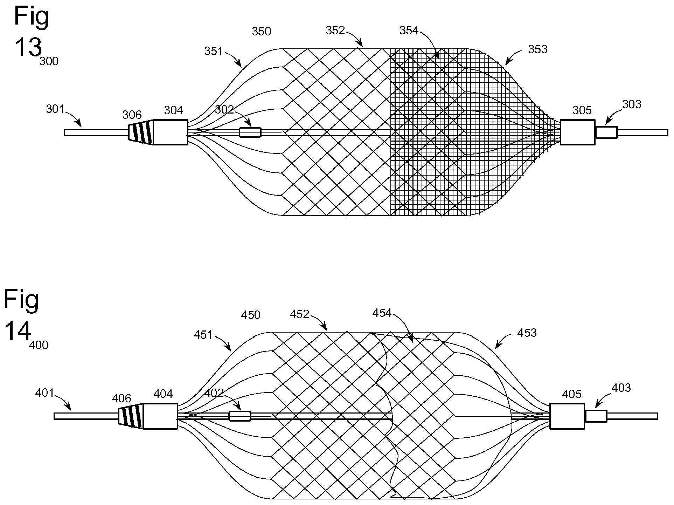

In another situation, the deformable element 250 the additional or optional purpose of filtering blood flowing in the blood vessel, such as for removing emboli dislodged from blood vessels upstream of the blood vessel. A filter of this type may be seen in figure 13, where the engagement portion 352 and the distal, tapering part 353 are provided with a filtering surface. This filtering surface may be an outer surface provided on a structure of the deformable element 350, such as a braid or the like. Alternatively, the filtering surface may be provided as a more dense part of a braid forming also the other parts of the deformable element but as a less dense portion more easily allowing both blood and emboli to pass. An advantage of providing the most distal portions of the deformable element with the filtering surface is that this surface will be inwardly tapering and thus will retain the filtered emboli when the deformable element is pulled into a retrieval catheter.

In figure 14, an alternative filter type may be seen where the filtering surface is provided as a separate, now inner, layer 454 provided inside the deformable element 450. The inner layer 454 may be attached to any of the tapering parts 453, 451 or the engagement portion 452. The inner layer may be a stretchable or non-stretchable layer made of e.g. a cloth (weaved, non-weaved) or a layer provided with holes/openings or a braid of e.g. nitinol wires. Again, the distally tapering shape will facilitate retaining of the emboli during retrieval.

An alternative to the filter is an occluder, which may be of the same type as seen in figures 13/14 where the filtering surface is replaced with an occluding surface. Occluding surfaces may be initially closed or may comprise a coagulating agent/surface which makes the blood coagulate there on and thereby form an occluding surface.

## Claims

1. An endovascular assembly (100) comprising:
- an elongate element (101, 201) having a proximal end (90) and a distal end (92),
- a deformable element (150, 250, 350, 450, 550) having a proximal slider (104, 204, 304, 404, 504) and a distal slider (105, 205, 305, 405, 505) configured to slide along the elongate element, the distal slider being positioned closer to the distal end than the proximal slider, the deformable element:
- having a rest shape where a first distance exists between the proximal slider and the distal slider along a predetermined direction, the deformable element, in the rest shape, defining a first circumscribed circle with a first radius, in a plane perpendicular to the predetermined direction,
- being extendable along the predetermined direction to a extended shape where a second distance exists between the proximal slider and the distal slider along the predetermined direction, the second distance exceeding the first distance, the deformable element, in the extended shape, defining a second circumscribed circle with a second radius, in the plane perpendicular to the predetermined direction, the first radius exceeding the second radius,
- being compressible along the predetermined direction to a compressed shape where a third distance exists between the proximal slider and the distal slider along the predetermined direction, the first distance exceeding the third distance, the deformable element, in the compressed shape, defining a third circumscribed circle with a third radius, in the plane perpendicular to the predetermined direction, the third radius exceeding the first radius,
**characterized in that** the assembly further comprises:
- a proximal stopper (102, 202, 302, 402, 502) fixed or fixable in relation to the elongate element, and
- a distal stopper (103, 203, 303, 403, 503) fixed or fixable in relation to the elongate element,
where:
- the distal stopper is positioned closer to the distal end than the proximal stopper,
- the proximal stopper is positioned closer to the distal end than the proximal slider, the proximal stopper being configured to prevent the proximal slider from moving closer to the distal end than the proximal stopper,
- the distal stopper is positioned closer to the distal end than the distal slider, the distal stopper being configured to prevent the distal slider from moving closer to the distal end than the distal stopper, and
- a distance, along the elongate element, between the proximal stopper and the distal stopper is no more than a predetermined distance being 0.95*the first distance.

2. The assembly according to claim 1, wherein the proximal and distal stoppers are fixedly attached in relation to the elongate element.

3. The assembly according to claim 1, wherein the proximal stopper is configured to pass the distal slider when moved distally.

4. The assembly according to claim 1, wherein the proximal stopper is configured to engage the distal slider when moved distally.

5. The assembly according to any of the preceding claims wherein the third distance is no more than 0.95*the first distance.

6. The assembly according to any of the preceding claims, wherein the elongate element is a single elongate element.

7. The assembly according to any of claims 1-5, wherein the elongate element comprises a first (211, 211') and a second (201, 201') elongate elements each having a proximal end and a distal end, wherein:
- the proximal slider is configured to slide along the second elongate element,
- the distal slider is configured to slide along the first elongate element,
- the proximal stopper is fixed or fixable in relation to the second elongate element, and
- the distal stopper is fixed or fixable in relation to the first elongate element,
the assembly further comprising a locking element configured to lock the first elongate element to the second elongate element to maintain a predetermined relative longitudinal relation, along the predetermined direction, between the proximal and distal sliders.

8. The assembly according to claim 7, where the first and second elongate elements are co-extending at at least a portion of the lengths thereof, the locking element being positioned so as to lock the first elongated element to the second elongate element at a position within the portion of the lengths.

9. The assembly according to claim 8, wherein, at the portion of the lengths, the first elongate element extends within the second elongate element.

10. The assembly according to any of claims 7-9, wherein the locking element is provided at the proximal end of at least one of the first and second elongate elements.

11. The assembly according to any of claims 7-10, further comprising an element (213) configured to define a minimum distance, along the elongate element, between the proximal stopper and the distal stopper.

## Patentansprüche

1. Endovaskuläre Anordnung (100), umfassend:
- ein langgestrecktes Element (101, 201), das ein proximales Ende (90) und ein distales Ende (92) aufweist,
- ein verformbares Element (150, 250, 350, 450, 550), das einen proximalen Schieber (104, 204, 304, 404, 504) und einen distalen Schieber (105, 205, 305, 405, 505) aufweist, die konfiguriert sind, um entlang des langgestreckten Elements zu gleiten, wobei der distale Schieber näher an dem distalen Ende als der proximale Schieber positioniert ist, wobei das verformbare Element:
- eine Ruheform aufweist, bei der ein erster Abstand zwischen dem proximalen Schieber und dem distalen Schieber entlang einer vorbestimmten Richtung besteht, wobei das verformbare Element, in der Ruheform, einen ersten umschriebenen Kreis mit einem ersten Radius in einer Ebene senkrecht zu der vorbestimmten Richtung definiert,
- entlang der vorbestimmten Richtung zu einer ausgedehnten Form ausdehnbar ist, wobei ein zweiter Abstand zwischen dem proximalen Schieber und dem distalen Schieber entlang der vorbestimmten Richtung besteht, wobei der zweite Abstand den ersten Abstand überschreitet, wobei das verformbare Element, in der ausgedehnten Form, einen zweiten umschriebenen Kreis mit einem zweiten Radius in der Ebene senkrecht zu der vorbestimmten Richtung definiert, wobei der erste Radius den zweiten Radius überschreitet,
- entlang der vorbestimmten Richtung zu einer zusammengedrückten Form zusammendrückbar ist, wobei ein dritter Abstand zwischen dem proximalen Schieber und dem distalen Schieber entlang der vorbestimmten Richtung besteht, wobei der erste Abstand den dritten Abstand überschreitet, wobei das verformbare Element, in der zusammengedrückten Form einen dritten umschriebenen Kreis mit einem dritten Radius in der Ebene senkrecht zu der vorbestimmten Richtung definiert, wobei der dritte Radius den ersten Radius überschreitet,
**dadurch gekennzeichnet, dass** die Anordnung ferner umfasst:
- ein proximales Anschlagelement (102, 202, 302, 402, 502), das in Bezug auf das langgestreckte Element befestigt oder befestigbar ist, und
- ein distales Anschlagelement (103, 203, 303, 403, 503), das in Bezug auf das langgestreckte Element befestigt oder befestigbar ist,
wobei:
- das distale Anschlagelement näher an dem distalen Ende positioniert ist als das proximale Anschlagelement,
- das proximale Anschlagelement näher an dem distalen Ende als der proximale Schieber positioniert ist, wobei das proximale Anschlagelement konfiguriert ist, um zu verhindern, dass sich der proximale Schieber näher an das distale Ende als das proximale Anschlagelement bewegt,
- das distale Anschlagelement näher an dem distalen Ende als der distale Schieber positioniert ist, wobei das distale Anschlagelement konfiguriert ist, um zu verhindern, dass sich der distale Schieber näher an das distale Ende als das proximale Anschlagelement bewegt, und
- ein Abstand entlang des langgestreckten Elements zwischen dem proximalen Anschlagelement und dem distalen Anschlagelement nicht größer als ein vorbestimmter Abstand ist, der das 0,95-fache des ersten Abstands ist.

2. Anordnung nach Anspruch 1, wobei die proximalen und distalen Anschlagelemente in Bezug auf das langgestreckte Element fest angebracht sind.

3. Anordnung nach Anspruch 1, wobei das proximale Anschlagelement konfiguriert ist, um den distalen Schieber zu passieren, wenn es in distaler Richtung bewegt wird.

4. Anordnung nach Anspruch 1, wobei das proximale Anschlagelement konfiguriert ist, in den distalen Schieber einzugreifen, wenn es in distaler Richtung bewegt wird.

5. Anordnung nach einem der vorhergehenden Ansprüche, wobei der dritte Abstand nicht größer als das 0,95-fache des ersten Abstands ist.

6. Anordnung nach einem der vorhergehenden Ansprüche, wobei das langgestreckte Element ein einzelnes langgestrecktes Element ist.

7. Anordnung nach einem der Ansprüche 1 bis 5, wobei das langgestreckte Element ein erstes (211, 211') und ein zweites (201, 201') langgestrecktes Element umfasst, die jeweils ein proximales Ende und ein distales Ende aufweisen, wobei:
- der proximale Schieber konfiguriert ist, um entlang des zweiten langgestreckten Elements zu gleiten,
- der distale Schieber konfiguriert ist, um entlang des ersten langgestreckten Elements zu gleiten,
- das proximale Anschlagelement in Bezug auf das zweite langgestreckte Element befestigt oder befestigbar ist, und
- das distale Anschlagelement in Bezug auf das erste langgestreckte Element befestigt oder befestigbar ist,
wobei die Anordnung ferner ein Verriegelungselement umfasst, das konfiguriert ist, um das erste langgestreckte Element mit dem zweiten langgestreckten Element zu verriegeln, um eine vorbestimmte relative Längenbeziehung entlang der vorbestimmten Richtung zwischen dem proximalen und dem distalen Schieber aufrechtzuerhalten.

8. Anordnung nach Anspruch 7, wobei das erste und das zweite langgestreckte Element sich mindestens mit einem Teilbereich der Längen davon gemeinsam ausdehnen, wobei das Verriegelungselement so positioniert ist, dass es das erste langgestreckte Element mit dem zweiten langgestreckten Element in einer Position innerhalb des Teilbereichs der Längen verriegelt.

9. Anordnung nach Anspruch 8, wobei sich das erste langgestreckte Element, in dem Teilbereich der Längen, innerhalb des zweiten langgestreckten Elements erstreckt.

10. Anordnung nach einem der Ansprüche 7 bis 9, wobei das Verriegelungselement an dem proximalen Ende von mindestens einem von dem ersten und dem zweiten langgestreckten Element vorgesehen ist.

11. Anordnung nach einem der Ansprüche 7 bis 10, ferner umfassend ein Element (213), das konfiguriert ist, um einen Mindestabstand entlang des langgestreckten Elements zwischen dem proximalen Anschlagelement und dem distalen Anschlagelement zu definieren.

## Revendications

1. Ensemble endovasculaire (100) comprenant :
- un élément allongé (101, 201) ayant une extrémité proximale (90) et une extrémité distale (92),
- un élément déformable (150, 250, 350, 450, 550) ayant une glissière proximale (104, 204, 304, 404, 504) et une glissière distale (105, 205, 305, 405, 505) configurées pour coulisser le long de l'élément allongé, la glissière distale étant positionnée plus proche de l'extrémité distale que la glissière proximale, l'élément déformable :
- ayant une forme au repos où une première distance existe entre la glissière proximale et la glissière distale le long d'une direction prédéterminée, l'élément déformable, dans la forme au repos, définissant un premier cercle circonscrit avec un premier rayon, dans un plan perpendiculaire à la direction prédéterminée,
- étant extensible le long de la direction prédéterminée en une forme étendue où une deuxième distance existe entre la glissière proximale et la glissière distale dans la direction prédéterminée, la deuxième distance excédant la première distance, l'élément déformable, dans la forme étendue, définissant un deuxième cercle circonscrit avec un deuxième rayon, dans le plan perpendiculaire à la direction prédéterminée, le premier rayon étant supérieur au deuxième rayon,
- étant compressible le long de la direction prédéterminée en une forme comprimée où une troisième distance existe entre la glissière proximale et la glissière distale le long de la direction prédéterminée, la première distance étant supérieure à la troisième distance, l'élément déformable, dans la forme comprimée, définissant un troisième cercle circonscrit avec un troisième rayon dans le plan perpendiculaire à la direction prédéterminée, le troisième rayon étant supérieur au premier rayon,
**caractérisé en ce que** l'ensemble comprend en outre :
- une butée proximale (102, 202, 302, 402, 502) fixée ou fixable par rapport à un élément allongé, et
- une butée distale (103, 203, 303, 403, 503) fixée ou fixable par rapport à un élément allongé,
où :
- la butée distale est positionnée plus proche de l'extrémité distale que la butée proximale,
- la butée proximale est positionnée plus proche de l'extrémité distale que la glissière proximale, la butée proximale étant configurée pour empêcher la glissière proximale de se rapprocher davantage de l'extrémité distale que de la butée proximale,
- la butée distale est positionnée plus proche de l'extrémité distale que la glissière distale, la butée distale étant configurée pour empêcher la glissière distale de se rapprocher davantage de l'extrémité distale que de la butée distale, et
- une distance, le long de l'élément allongé, entre la butée proximale et la butée distale n'est pas supérieure à une distance prédéterminée étant 0,95 x la première distance.

2. Ensemble selon la revendication 1, dans lequel les butées proximale et distale sont solidement fixées par rapport à l'élément allongé.

3. Ensemble selon la revendication 1, dans lequel la butée proximale est configurée pour passer la glissière distale lorsqu'elle est distalement déplacée.

4. Ensemble selon la revendication 1, dans lequel la butée proximale est configurée pour se mettre en prise avec la glissière distale lorsqu'elle est déplacée distalement.

5. Ensemble selon l'une quelconque des revendications précédentes dans lequel la troisième distance n'est pas supérieure à 0,95 x la première distance.

6. Ensemble selon l'une quelconque des revendications précédentes, dans lequel l'élément allongé est un élément allongé unique.

7. Ensemble selon l'une quelconque des revendications 1 à 5, dans lequel l'élément allongé comprend un premier (211, 211') et un second (201, 201') élément allongé ayant chacun une extrémité proximale et une extrémité distale, dans lequel :
- la glissière proximale est configurée pour coulisser le long du second élément allongé,
- la glissière distale est configurée pour coulisser le long du premier élément allongé,
- la butée proximale est fixée ou fixable par rapport au second élément allongé, et
- la butée distale est fixée ou fixable par rapport au premier élément allongé,
l'ensemble comprenant en outre un élément de verrouillage configuré pour verrouiller le premier élément allongé au second élément allongé pour maintenir une relation longitudinale relative prédéterminée, dans la direction prédéterminée, entre les glissières proximale et distale.

8. Ensemble selon la revendication 7, dans lequel les premier et second éléments allongés sont coextensifs au niveau d'au moins une partie de leurs longueurs, l'élément de verrouillage étant positionné de manière à verrouiller le premier élément allongé au second élément allongé au niveau d'une position à l'intérieur de la partie des longueurs.

9. Ensemble selon la revendication 8, dans lequel, au niveau de la partie des longueurs, le premier élément allongé s'étend à l'intérieur du second élément allongé.

10. Ensemble selon l'une quelconque des revendications 7 à 9, dans lequel l'élément de verrouillage est fourni au niveau de l'extrémité proximale d'au moins l'un des premier et second éléments allongés.

11. Ensemble selon l'une quelconque des revendications 7 à 10, comprenant en outre un élément (213) configuré pour définir une distance minimale le long de l'élément allongé, entre la butée proximale et la butée distale.
